# EUROPÄISCHE PATENTSCHRIFT

(11) **EP 0 649 830 B1**
(45) Veröffentlichungstag und Bekanntmachung des Hinweises auf die Patenterteilung: **21.01.1998**
(21) Anmeldenummer: 94116237.2
(22) Anmeldetag: 14.10.1994
(51) Int. Cl.: C07C 46/06, C07C 50/24

(54) **Verfahren zur Herstellung von Tetrachlor-1,4-benzochinon**
Process for the preparation of tetrachloro-1,4-Benzoquinone
Procédé pour la préparation de la tétrachloro-1,4-benzoquinone

(30) Priorität: 25.10.1993 DE 4336323
(43) Veröffentlichungstag der Anmeldung: 26.04.1995
(73) Patentinhaber: Clariant GmbH, 65929 Frankfurt am Main (DE)
(72) Erfinder: Arndt, Otto, Dr., D-65719 Hofheim (DE); Schubert, Hans, Dr., D-65779 Kelkheim (DE)

(56) Entgegenhaltungen:
- EP-A- 0 220 135
- EP-A- 0 278 378
- DATABASE WPI Section Ch, Week 9346, Derwent Publications Ltd., London, GB; Class E14, AN 93-365183 & JP-A-5 271 144 (TOKUYAMA SODA KK) 19. Oktober 1993

## Beschreibung

Die Erfindung betrifft ein Verfahren zur Herstellung von Tetrachlor-1,4-benzochinon hoher Reinheit durch Chlorierung von Hydrochinon

Tetrachlor-1,4-benzochinon (Chloranil) ist ein wertvolles Zwischenprodukt zur Herstellung von Farbstoffen und Schädlingsbekämpfungsmitteln. Ferner wird es als Photochemikalie und Vulkanisationsmittel eingesetzt und dient als Zusatz für Schmiermittel.

Die Herstellung von Chloranil aus Hydrochinon (1,4-Dihydroxy-benzol) oder 1,4-Benzochinon bzw. chloriertem 1,4-Benzochinon ist bekannt.

In der Patentschrift EP 0 220 135 wird z. B. ein Verfahren beschrieben, bei dem Salzsäure und Chlor unter Druck vorgelegt werden und Chlor und Chinon, Hydrochinon oder deren Chlorderivate in getrennten Strömen unter Druck zudosiert werden.

In Patentschrift EP 0 278 378 wird ein Verfahren beschrieben, bei dem die Gesamtmenge des Hydrochinons mit Salzsäure vorgelegt wird und dann Chlor bei Normaldruck nach einem bestimmten Temperatur- und Verdünnungsprogramm eingegast wird.

Beide Verfahrensweisen weisen Nachteile auf:

Das Verfahren nach EP 0 220 135 verwendet 3 bis 12 bar Chlordruck und erfordert eine exakte synchronisierte Dosierung für die Chlor- und Hydrochinon-Ströme.

Das Verfahren nach EP 0 278 378 hat folgende Nachteile:
1.) Durch das Vorlegen der Gesamtmenge des Hydrochinons bilden sich im Laufe der Reaktion Krusten an der Reaktorwand. Außerdem durchläuft das Reaktionsgemisch Zustände schlechter Rührbarkeit, da es dabei sehr dick wird.
2.) Die langanhaltende thermische Belastung des Reaktionsgemischs bei Temperaturen oberhalb 100°C bewirkt eine Qualitätsverschlechterung durch Spuren Nebenprodukte.
3.) Die lange Nachchlorierungsdauer bedingt ein Verschlechtern der Raumzeitausbeute.

Es besteht daher ein Bedarf nach einem einfachen und technisch leicht durchführbaren Verfahren zur Herstellung von Tetrachlor-1,4-benzochinon, das die vorstehend genannten Nachteile vermeidet und Tetrachlor-1,4-benzochinon nicht nur in hoher Reinheit, sondern auch in hoher Ausbeute und Raumzeitausbeute zugänglich macht.

Diese Aufgabe wird gelöst durch ein Verfahren zur Herstellung von Tetrachlor-1,4-benzochinon hoher Reinheit durch Einwirkung von Chlor und konzentrierter Salzsäure auf Hydrochinon, dadurch gekennzeichnet, daß man einen Teil des einzusetzenden Hydrochinons in vorgelegte, katalytische Mengen Eisen(III)-Ionen und ein anionisches Dispergiermittel enthaltende 4 bis 6 fach molare Menge - bezogen auf die Gesamtmenge Hydrochinon - 20 bis 37 % wäßrige Salzssäure einträgt, in diese Lösung bei einer Temperatur von 20 bis 90°C die 1,5 bis 2,0fach molare Menge - bezogen auf Gesamthydrochinon - Chlorgas einleitet, anschließend die restliche Menge Hydrochinon als Feststoff oder gelöst zugibt, die 1,5 bis 2,0 fach molare Chlorgasmenge einleitet, hierbei durch Zugabe von Wasser die Konzentration der Salzsäure bei 23-25 % hält und schließlich unter weiterem Chloreingasen (1,7 bis 2,5fach molare Menge) und Verdünnen mit Wasser bis zu einer Salzsäurekonzentration von 20 bis 22 % die Temperatur bis auf 100 bis 107°C steigert.

In vielen Fällen hat es sich bewährt, 22 bis 31 %ige Salzsäure einzusetzen und die erste Phase der Chlorierung bei einer Temperatur von 40 bis 90°C, insbesondere 50 bis 80°C durchzuführen. Es hat sich als vorteilhaft erwiesen, 30 bis 70 %, insbesondere 40 bis 60 %, bevorzugt 45 bis 55 % des einzusetzenden Hydrochinons vorzulegen und die Konzentration der Salzsäure auf 22 bis 28 %, insbesondere 23 bis 25 %, einzustellen.

Die Chlorierung läuft vorteilhaft in 5 bis 15 Stunden, insbesondere 8,5 bis 10,5 Stunden ab.

Durch diese Verfahrensweise lassen sich gegenüber EP 0 278 378 überraschenderweise wesentliche Verbesserungen von Raumzeitausbeute und Produktqualität erreichen. Die Verfahrensänderung besteht darin, daß nicht mehr die Gesamtmenge des Hydrochinons vorgelegt wird, sondern daß das Hydrochinon in zwei Anteilen dem Ansatz zugegeben wird: der erste Teil als Vorlage zusammen mit der Salzsäure, der zweite Teil als Feststoff oder in wäßriger ca. 10 bis 20 %iger, insbesondere 15-18 %iger Lösung in einmaliger Zugabe bei erhöhter Temperatur nach dem ersten Einleiten von Chlorgas. Dabei läuft Wasser in konstantem Strom zu, so daß die Konzentration der Salzsäure auf ca. 23-25 % gehalten wird. Nach Ablauf der ersten Chlorierungsstufe (Bildung der polychlorierten Chinhydrone) wird weiter Chlor eingegast, jedoch nun unter Temperaturerhöhung bis nahe an den Siedepunkt der azeotropen Salzsäure (ca. 105 bis 107°C), deren Konzentration von ca. 20 % durch Wasserzugabe eingestellt wird. In dieser Phase dient das Chlor neben der Einführung des vierten Cl-Atoms in den Kern auch in verstärktem Maße zur Oxidation der chlorierten Hydrochinone bzw. Chinhydrone, wobei sich die Gegenwart von Spuren Eisen(III)-Ionen als Oxidationskatalysator bewährt hat.

Falls erforderlich kann die Qualität des isolierten Chloranils noch durch Nachbehandlung mit siedendem Wasser verbessert werden.

Die Vorteile dieser Verfahrensweise gegenüber EP 0 278 378 sind: Die technische Durchführbarkeit der Reaktion wird verbessert: Die Rührbarkeit wird erleichtert trotz verringertem Salzsäure-Einsatz, der Wärmeübergang verbessert, die Kühlsole entfällt, die Homogenität des Reaktionsgemischs wird durch den Wegfall der Krusten hergestellt, der Zeitaufwand für die Chlorierung und Oxidation werden erniedrigt (raschere Oxidation, bessere Abreaktion von Restmengen an Trichlorbenzochinon), die Einsatzmenge des Chlorgases und damit auch die Abgasbelastung an Chlor wird verringert, die Qualität des Produkts (ersichtlich an weniger Komponenten im HPLC) und ihre Reproduzierbarkeit werden verbessert.

Das Verfahren ist flexibel in der Temperaturführung und in der Art der Zugabe des zweiten Anteils des Hydrochinons.

Die Gründe für die gegenüber dem zitierten Stand der Technik verbesserte Verfahrensführung und verbesserte Reproduzierbarkeit sind
a) das Arbeiten bei Normaldruck (Aspekte der Sicherheit),
b) die einfacheren Dosierbedingungen (eine vorübergehende Chlorüberdosierung ist unschädlich bezüglich Abgasbelastung, da das Chlor durch das im Überschuß vorliegende Hydrochinon abgepuffert wird). Der 2. Anteil des Hydrochinons kann als Feststoff oder als wäßrige Lösung schnell vor weiterem Chlor-Eingasen oder langsam mit dem weiterem Chlor-Eingasen zugegeben werden.
c) die einfachere Kühltechnik (Kühlung auf höherem Temperaturniveau),
d) die bessere Rührbarkeit der Suspension trotz verringerter Salzsäurevorlage,
e) die geringere Neigung zur Krustenbildung,
f) die Abnahme von Chlor im Abgas,
g) kein Chlorwasserstoff im Abgas,
h) die raschere Oxidation der intermediär gebildeten polychlorierten Chinhydrone bzw. Hydrochinone durch das Chlor,
i) Die geringere thermische Belastung bei der Nachchlorierung,
j) die Unabhängigkeit von der Zusammensetzung der Gasatmosphäre im Reaktor (Luft oder Stickstoff),
k) die konstante, eine Umkristallisation vermeidende Qualität des isolierten Chloranils.

Das erfindungsgemäß hergestellte Chloranil ist von hoher Reinheit, (Schmelzpunkt, HPLC) und hat im Vergleich zu den Angaben in der zitierten EP 0 278 378 weniger Nebenkomponenten bei gleichzeitig erhöhter Raumzeitausbeute.

Die nachstehenden Beispiele dienen der Erläuterung des erfindungsgemäßen Verfahrens, ohne es zu beschränken. Teile sind hierbei Gewichtsteile.

### Beispiel 1

### Erste Stufe

Man trägt unter Luftatmosphäre in 287 Teile Salzsäure 31 % (2,4 Mol), die 0,024 Teile Eisen(III)chlorid als Oxidationskatalysator und 0,50 Teile eines anionischen Dispergiermittels enthält, 27,65 Teile Hydrochinon (0,25 Mol) als Feststoff ein. Man gast bei 55°C in 3 Stunden 125 Teile Chlor (1,76 Mol) ein. Gleichzeitig laufen 250 Teile 55°C warmes Wasser zu. Wenn 50 % des Chlors und des Wassers zugegeben sind, werden 27,65 Teile Hydrochinon (0,25 Mol) als Feststoff in einer einmaligen Portion zugegeben. Die gemäß HPLC über alle Chlorierungsstufen verlaufende Reaktion wird bereits jetzt von der zum chlorierten Benzochinon führenden Oxidation (Oxidationsmittel = Chlor) begleitet. Man erhält eine gut rührbare, krustenfreie, gelbbraune Suspension. Das Abgas wird überwacht. Es dürfen kein Chlor und kein Chlorwasserstoff in das Abgas gelangen. Die Konzentration der Salzsäure beträgt ca. 25 %.

### Zweite Stufe

Das Gemisch aus polychlorierten Benzochinonen und Hydrochinonen (bzw. Chinhydronen) wird in 3 Stunden auf 85°C unter weiterem Eingasen von 50 Teilen Chlor (= 0,70 Mol) geheizt. Nachdem 50 % des Chlors eingegast sind, läßt man 250 Teile Wasser zulaufen. Die Konzentration der Salzsäure beträgt vor dem Verdünnen ca. 27 %, danach ca. 20 %. In der zweiten Stufe wird die Oxidation vervollständigt. Man erhält eine gut rührbare, krustenfreie, gelbe Suspension von Tetrachlorbenzochinon, die noch etwas Trichlorbenzochinon enthält. Das Abgas wird überwacht. Es darf nur wenig Chlor (max. 7 Teile = 0,1 Mol) ins Abgas gelangen.

### Dritte Stufe

Die Suspension wird in ca. 1 Stunde auf 105°C geheizt. Dabei darf noch kein Chlorwasserstoff entweichen.

### Vierte Stufe

Die Temperatur des Reaktionsgemischs wird 3 Stunden auf 103 bis 106°C, bevorzugt 105°C, gehalten. Dabei wird erneut Chlor eingegast (max. 25 Teile = 0,35 Mol). Die Oxidation von restlichem Tetrachlorhydrochinon und die Vervollständigung der Chlorierung von restlichem Trichlorbenzochinon zum Tetrachlorbenzochinon (= Chloranil) werden mit DC und HPLC verfolgt. Das Abgas enthält jetzt ca. 7 Teile Chlor (= ca. 0,1 Mol). Man arbeitet in allen Stufen im offenen System (Belüftung über Abgasanlage), so daß sich kein Druck aufbauen kann. Die Chlorierzeit beträgt max. 10 Stunden. Insgesamt werden 200 Teile Chlor (2,82 Mol) eingegast. Man kühlt den Ansatz auf 40°C unter Überlagerung mit Schutzgas (Stickstoff) ab, wodurch ein Zurücksaugen von Chlor aus der Abgasabsorption verhindert wird und die Atmosphäre über dem Reaktionsgemisch entgiftet wird. Nach Filtration bei 40°C und Waschen mit 500 Teilen Wasser erhält man 119 Teile gelbes Chloranil mit einem Reingehalt von 98 % (0,474 Mol), entsprechend einer Ausbeute von 95 %. Tetrachlorhydrochinon bzw. andere polychlorierte Chinhydrone bzw. Hydrochinone sind nicht nachweisbar (DC). Der Gehalt an 2,3,5-Trichlorbenzochinon beträgt max. 2,0 Gew.-% (HPLC).

Die Mutterlauge (795 Teile) ist eine ca. 21 %ige Salzsäure (4,6 Mol HCl), das Waschfiltrat (503 Teile) enthält ca. 4,4 % = 0,6 Mol HCl. Die Chlorbilanz (Chlorwasserstoff und Chlor, incl. Abgas) beträgt ca. 95 % der Theorie. Das umgesetzte Chlor entspricht der Theorie. Zu Beginn der vierten Stufe liegen noch geringe Mengen Tetrachlorhydrochinon vor (DC).

### Beispiel 2

### Erste Stufe

Man erfährt wie in Beispiel 1, jedoch bei einer Temperatur von 70°C und mit nur 125 Teilen Wasser. Die Zugabe des zweiten Anteils Hydrochinon erfolgt als ca. 18 %ige wäßrige Lösung von 70°C (mit 125 Teilen Wasser) entweder rasch vor dem zweiten Anteil Chlor oder gleichzeitig mit dem zweiten Anteil Chlor. In dieser Stufe fällt kein Abgas an.

### Zweite Stufe

Bei 70°C (Beibehaltung der Temperatur der ersten Stufe) werden in 2 Stunden 45 Teile Chlor (0,63 Mol) eingegast. Dann läßt man 200 Teile Wasser zulaufen. Die Konzentration der Salzsäure beträgt vor dem Verdünnen ca. 28 %, danach ca. 21 %. In der zweiten Stufe wird die Oxidation vervollständigt.

### Dritte Stufe

Man heizt in 3 Stunden auf 105°C unter weiterem Eingasen von 25 Teilen Chlor (0,35 Mol). Die Addition von HCl an Trichlorbenzochinon wird vermieden. Das Abgas aus der 2, und 3. Stufe enthält 23 Teile Chlor.

### Vierte Stufe

Die Temperatur wird 2 Stunden bei 103 bis 106°C, bevorzugt 105°C, gehalten. Dabei werden max. 15 Teile (0,21 Mol) Chlor eingegast. Das Abgas enthält 10 Teile Chlor. Die gesamte Chlorierungszeit (über alle Stufen) beträgt 10 Stunden. Insgesamt werden 210 Teile Chlor eingegast. Man erhält 120 Teile Chloranil mit einem Reingehalt von 98,3 % (0,480 Mol), entsprechend einer Ausbeute von 96 %. Tetrachlohydrochinon bzw. andere polychlorierte Chinhydrone bzw. Hydrochinone sind nicht nachweisbar (DC). Der Gehalt an 2,3,5-Trichlorbenzochinon beträgt max. 1,7 Gew.-% (HPLC), der Gehalt an Pentachlorphenol ca. 100 µg/g. Die Chlorbilanz (Chlorwasserstoff und Chlor, incl. Abgas) beträgt ca. 100 % d. Th.. Der Chlorumsatz beträgt 99,4 % d. Th.. Zu Beginn der dritten Stufe liegt kein polychloriertes Hydrochinon bzw. Chinhydron mehr vor.

### Beispiel 3

### (Nachbehandlung mit Wasser)

30 Teile eines Chloranilmusters mit dem Schmelzpunkt von 270 bis 274°C, einem dünnschichtchromatographisch noch in Spuren nachweisbarem Gehalt von polychlorierten Chinhydronen bzw. Hydrochinonen und einem Gehalt an Pentachlorphenol von 120 µg/g werden in 125 Teilen Wasser 2 Stunden bei 100°C behandelt und dann bei 90°C filtriert. Man erhält 29 Teile Chloranil mit dem Schmelzpunkt 293 bis 295°C, einem dünnschichtchromatographisch nicht mehr nachweisbarem Gehalt von polychlorierten Chinhydronen bzw. Hydrochinonen und einem Gehalt an Pentachlorphenol von kleiner 20 µg/g.

## Patentansprüche

1. Verfahren zur Herstellung von Tetrachlor-1,4-benzochinon hoher Reinheit durch Einwirkung von Chlor und konzentrierter Salzsäure auf Hydrochinon, dadurch gekennzeichnet, daß man einen Teil des einzusetzenden Hydrochinons in vorgelegte, katalytische Mengen Eisen(III)-Ionen und ein anionisches Dispergiermittel enthaltende 4 bis 6 fach molare Menge - bezogen auf die Gesamtmenge Hydrochinon - 20 bis 37 % wäßrige Salzssäure einträgt, in diese Lösung bei einer Temperatur von 20 bis 90°C die 1,5 bis 2,0 fach molare Menge - bezogen auf Gesamthydrochinon - Chlorgas einleitet, anschließend die restliche Menge des Hydrochinons als Feststoff oder gelöst zugibt, die 1,5 bis 2,0 fach molare Chlorgasmenge einleitet, hierbei durch Zugabe von Wasser die Konzentration der Salzsäure bei 23-25 % hält und schließlich unter weiterem Chloreingasen (1,7 bis 2,5 fach molare Menge) und Verdünnen mit Wasser bis zu einer Salzsäurekonzentration von 20 bis 22 % die Temperatur bis auf 100 bis 107°C steigert.

2. Verfahren nach Anspruch 1, dadurch gekennzeichnet, daß man 30 bis 70 %, insbesondere 40 bis 60 %, bevorzugt 45 bis 55 % des einzusetzenden Hydrochinons vorlegt.

3. Verfahren nach Anspruch 1 oder 2, dadurch gekennzeichnet, daß die eingesetzte wäßrige Salzsäure 22 bis 28 %ig ist, insbesondere 23 bis 25 %ig ist.

4. Verfahren nach mindestens einem der Ansprüche 1 bis 3, dadurch gekennzeichnet, daß bei einer Temperatur von 40 bis 90°C, insbesondere 50 bis 80°C Chlor eingegast wird.

5. Verfahren nach mindestens einem der Ansprüche 1 bis 4, dadurch gekennzeichnet, daß die restliche Menge des Hydrochinons als 10 bis 20 %ige, insbesondere 15-18 %ige wäßrige Lösung zugegeben wird.

6. Verfahren nach mindestens einem der Ansprüche 1 bis 5, dadurch gekennzeichnet, daß die Temperatur beim dritten Chloreingasen auf 103 bis 106°C, insbesondere 104 bis 105°C gesteigert wird.

7. Verfahren nach mindestens einem der Ansprüche 1 bis 6, dadurch gekennzeichnet, daß die Chlorierung in 5 bis 15 Stunden, insbesondere 8,5 bis 10,5 Stunden durchgeführt wird.

8. Verfahren nach mindestens einem der Ansprüche 1 bis 7, dadurch gekennzeichnet, daß man das isolierte Chloranil noch einer Nachbehandlung mit siedendem Wasser unterzieht.

## Claims

1. A process for the preparation of tetrachloro-1,4-benzoquinone of high purity by the action of chlorine and concentrated hydrochloric acid on hydroquinone, which comprises introducing a part of the hydroquinone to be employed into an initially introduced 4- to 6-fold molar amount - based on the total amount of hydroquinone - of 20 to 37 % aqueous hydrochloric acid containing catalytic amounts of iron(III) ions and an anionic dispersant, introducing 1.5 to 2.0 times the molar amount - based on total hydroquinone - of chlorine as a gas into this solution at a temperature of 20 to 90°C, then adding the residual amount of hydroquinone as a solid or in dissolved form, introducing 1.5 to 2.0 times the molar amount of chlorine as a gas, keeping the concentration of the hydrochloric acid here at 23-25 % by addition of water and finally raising the temperature to 100 to 107°C with further introduction of chlorine as a gas (1.7 to 2.5 times the molar amount) and dilution with water to a hydrochloric acid concentration of 20 to 22 %.

2. The process as claimed in claim 1, wherein 30 to 70%, in particular 40 to 60 %, preferably 45 to 55%, of the hydroquinone to be employed is initially introduced.

3. The process as claimed in claim 1 or 2, wherein the aqueous hydrochloric acid employed is 22 to 28 % strength, in particular 23 to 25 % strength.

4. The process as claimed in at least one of claims 1 to 3, wherein chlorine is introduced as a gas at a temperature of 40 to 90°C, in particular 50 to 80°C.

5. The process as claimed in at least one of claims 1 to 4, wherein the residual amount of hydroquinone is added as a 10 to 20 % strength, in particular 15-18 % strength, aqueous solution.

6. The process as claimed in at least one of claims 1 to 5, wherein the temperature in the third introduction of chlorine as a gas is raised to 103 to 106°C, in particular 104 to 105°C.

7. The process as claimed in at least one of claims 1 to 6, wherein the chlorination is carried out in the course of 5 to 15 hours, in particular 8.5 to 10.5 hours.

8. The process as claimed in at least one of claims 1 to 7, wherein the isolated chloranil is additionally subjected to a subsequent treatment with boiling water.

## Revendications

1. Procédé pour la préparation de la tétrachloro-1,4-benzoquinone avec une pureté élevée par l'action du chlore et de l'acide concentré sur l'hydroquinone, caractérisé en ce que l'on introduit une partie de l'hydroquinone à utiliser dans une quantité 4 à 6 fois en moles - par rapport à la quantité totale d'hydroquinone - d'acide chlorhydrique aqueux à 20 à 37 %, contenant des quantités catalytiques d'ions fer(III) et un agent anioqie de mise en dispersion, on introduit dans cette solution, à une température de 20 à 90 °C, la quantité 1,5 à 2,0 fois molaire - par rapport à la totalité d'hydroquinone - de chlore gazeux, ensuite on ajoute la quantité restante d'hydroquinone sous forme solide ou dissoute, on introduit la quantité 1,5 à 2,0 fois molaire de chlore gazeux, ce faisant, on maintient la concentration d'acide chlorhydrique à 23 à 25 % par addition d'eau, et ensuite en introduisant encore du chlore gazeux (en quantité 1,7 à 2,5 fois molaire) et en diluant avec de l'eau jusqu'à une concentration d'acide chlorhydrique de 20 à 22 % en poids, on augmente la température jusqu'à 100 à 107 °C.

2. Procédé selon la revendication 1, caractérisé en ce que l'on prend 30 à 70 %, plus particulièrement 40 à 60 %, de préférence 45 à 55 % de l'hydroquinone à utiliser.

3. Procédé selon la revendication 1 ou 2, caractérisé en ce que l'on utilise l'acide chlorhydrique aqueux à une concentration de 22 à 28 %, plus particulièrement 23 à 25 %.

4. Procédé selon au moins l'une des revendications 1 à 3, caractérisé en ce que l'on introduit du chlore gazeux à une température de 40 à 90 °C, plus particulièrement à 50 à 80 °C.

5. Procédé selon au moins l'une des revendications 1 à 4, caractérisé en ce que l'on ajoute la quantité restante de l'hydroquinone sous forme de solution aqueuse à 10 à 20 %, plus particulièrement à 15 à 18 %.

6. Procédé selon au moins l'une des revendications 1 à 5, caractérisé en ce que l'on augmente la température lors de la troisième introduction du chlore gazeux à 103 à 106 °C, plus particulièrement à 104 à 105 °C.

7. Procédé selon au moins l'une des revendications 1 à 6, caractérisé en ce que l'on met en oeuvre la chloration en l'espace de 5 à 15 heures, plus particulièrement de 8,5 à 10,5 heures.

8. Procédé selon au moins l'une des revendications 1 à 7, caractérisé en ce que l'on traite le chloranile isolé encore par un post-traitement à l'eau bouillante.
